(19) Europäisches Patentamt
European Patent Office
Office européen des brevets

(11) Veröffentlichungsnummer: **0 300 313**
**A2**

(12) **EUROPÄISCHE PATENTANMELDUNG**

(21) Anmeldenummer: **88111036.5**

(22) Anmeldetag: **11.07.88**

(51) Int. Cl.4: **C07D 251/18 , C07D 251/50 , C07D 251/52 , C07D 251/54 , C07D 405/12 , A01N 43/68 , A01N 43/70**

(30) Priorität: **23.07.87 DE 3724378**
**16.01.88 DE 3801113**

(43) Veröffentlichungstag der Anmeldung:
**25.01.89 Patentblatt 89/04**

(84) Benannte Vertragsstaaten:
**BE CH DE ES FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Wroblowsky, Heinz-Jürgen, Dr.**
**Gladbacher Strasse 34**
**D-4018 Langenfeld(DE)**
Erfinder: **Kluth, Joachim, Dr.**
**Kurt-Schumacher-Strasse 9**
**D-4018 Langenfeld(DE)**
Erfinder: **Tietjen, Klaus-Günther, Dr.**
**Am Alten Broich 64a**
**D-4018 Langenfeld(DE)**
Erfinder: **Santel, Jans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Harry, Dr.**
**Unterdorfstrasse 6A**
**D-4000 Düsseldorf 31(DE)**

(54) **Substituierte Triazine.**

(57) Die Erfindung betrifft neue substituierte Triazine der allgemeinen Formel (I)

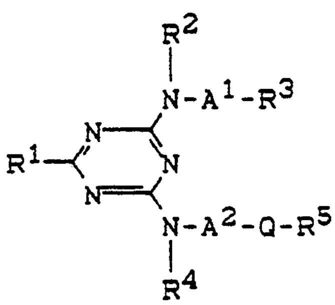

(I)

(in welcher $A^1$, $A^2$, Q, $R^1$, $R^2$, $R^3$, $R^4$ und $R^5$ die in der Beschreibung angegebenen Bedeutungen haben)

EP 0 300 313 A2

sowie Verfahren zu ihrer Herstellung und ihre Verwendung als Herbizide.

## Substituierte Triazine

Die Erfindung betrifft neue substituierte Triazine, Verfahren zu ihrer Herstellung und ihre Verwendung als Herbizide.

Es ist bekannt, daß bestimmte substituierte Triazine, wie z. B. 2-Chlor-4-ethylamino-6-isopropylamino-s-triazin (Atrazin), als Herbizide eingesetzt werden können (vgl. "Chemie der Pflanzenschutz- und Schädlingsbekämpfungsmittel", Band 5 - Herbizide, herausgegeben von R. Wegler, Springer-Verlag, Berlin, Heidelberg, New York 1977, S. 336 - 352). Die Wirkung der genannten Verbindung gegen hirseartige Unkräuter, wie z. B. Digitaria und Panicum, ist jedoch nicht immer ganz zufriedenstellend.

Es wurden nun neue substituierte Triazine der allgemeinen Formel (I)

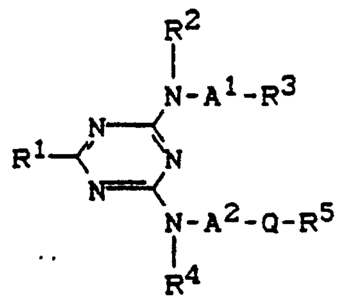

(I)

in welcher

$A^1$ für gegebenenfalls verzweigtes und gegebenenfalls durch Aryl substituiertes Alkandiyl ("Alkylen") steht,

$A^2$ für gegebenenfalls verzweigtes Alkandiyl ("Alkylen") steht,

Q für Sauerstoff, Schwefel, NH oder N-Alkyl steht,

$R^1$ für Wasserstoff, Hydroxy, Nitro, Cyano, Cyanamino, Azido, Halogen, Alkoxy, Alkylthio (welches gegebenenfalls durch Halogen oder Cyano substituiert ist), Alkyl, Alkoxycarbonyl, Aminocarbonyl, Alkylaminocarbonyl, Dialkylamino-carbonyl, Amino, Alkylamino oder Dialkylamino steht,

$R^2$ für Wasserstoff, Alkyl, Alkenyl oder Alkinyl steht,

$R^3$ für gegebenenfalls substituiertes Aryl oder Heteroaryl steht,

$R^4$ für Wasserstoff, Alkyl, Alkenyl oder Alkinyl steht und

$R^5$ für Wasserstoff oder Alkyl steht,

gefunden.

Weiter wurde gefunden, daß man die neuen substituierten Triazine der allgemeinen Formel (I) erhält, wenn man

(a) halogenierte Triazine der allgemeinen Formel (II)

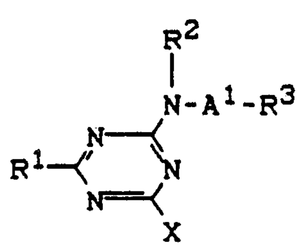

(II)

in welcher

$A^1$, $R^1$, $R^2$ und $R^3$ die oben angegebenen Bedeutungen haben und

X für Halogen steht,

mit Aminoverbindungen der allgemeinen Formel (III)

H-N- $A^2$-Q-$R^5$
$R^4$

(III)

in welcher

$A^2$, Q, $R^4$ und $R^5$ die oben angegebenen Bedeutungen haben,

gegebenenfalls in Gegenwart eines Säureakzeptors und gegebenenfalls in Gegenwart eines Verdünnungsmittels umsetzt, oder

(b) halogenierte Triazine der allgemeinen Formel (IV)

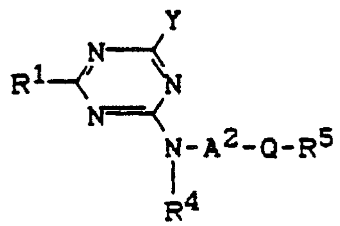

(IV)

in welcher

$A^2$, Q, $R^1$, $R^4$ und $R^5$ die oben angegebenen Bedeutungen haben und

Y für Halogen steht,

mit Aminoverbindungen der allgemeinen Formel (V)

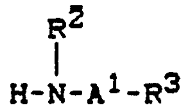

(V)

$A^1$, $R^2$ und $R^3$ die oben angegebenen Bedeutungen haben,

gegebenenfalls in Gegenwart eines Säureakzeptors und gegebenenfalls in Gegenwart eines Verdünnungsmittels umsetzt, oder

- für den Fall, daß $R^1$ für Amino, Alkylamino, Dialkylamino, Alkoxy oder Alkylthio steht -

(c) halogenierte Triazine der allgemeinen Formel (VI)

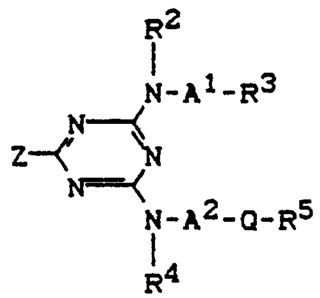

(VI)

in welcher

$A^1$, $A^2$, Q, $R^2$, $R^3$, $R^4$ und $R^5$ die oben angegebenen Bedeutungen haben und

Z für Halogen steht,

mit Ammoniak, Alkylaminen, Dialkylaminen, Alkanolen bzw. Alkanthiolen oder mit deren Alkalimetallsalzen gegebenenfalls in Gegenwart eines Säureakzeptors und gegebenenfalls in Gegenwart eines Verdünnungsmittels umsetzt, oder

- für den Fall, daß $R^1$ für Alkylthio (welches gegebenenfalls durch Halogen oder Cyano substituiert ist) steht -

(d) Mercaptotriazine der allgemeinen Formel (VII)

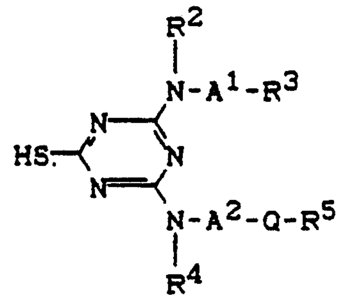

(VII)

in welcher
$A^1$, $A^2$, Q, $R^2$, $R^3$, $R^4$ und $R^5$ die oben angegebenen Bedeutungen haben,
mit Halogenverbindungen der allgemeinen Formel (VIII)
$Z^1 - R^6$ (VIII)
in welcher
$R^6$ für Alkyl (welches gegebenenfalls durch Halogen oder Cyano subsituiert ist) steht und
$Z^1$ für Halogen steht,
gegebenenfalls in Gegenwart eines Säureakzeptors und gegebenenfalls in Gegenwart eines Verdünnungsmittels umsetzt.

Die neuen substituierten Triazine der allgemeinen Formel (I) zeichnen sich durch starke herbizide Wirksamkeit aus.

Überraschenderweise zeigen die erfindungsgemäßen Verbindungen der allgemeinen Formel (I) bessere Selektivität in Kulturpflanzen und erheblich stärkere Wirkung gegen Unkräuter als nach Struktur und Wirkprofil vergleichbare bekannte Verbindungen.

Gegenstand der Erfindung sind vorzugsweise Verbindungen der Formel (I), in welcher
$A^1$ für gegebenenfalls verzweigtes und gegebenenfalls durch Phenyl substituiertes Alkandiyl ("Alkylen") mit 1 bis 4 Kohlenstoffatomen steht,
$A^2$ für gegebenenfalls verzweigtes Alkandiyl ("Alkylen") mit 1 bis 4 Kohlenstoffatomen steht,
Q für Sauerstoff, Schwefel, NH oder N-($C_1$-$C_4$-Alkyl) steht,
$R^1$ für Wasserstoff, Hydroxy, Nitro, Cyano, Cyanamino, Azido, Fluor, Chlor, Brom, Iod, $C_1$-$C_4$-Alkoxy, $C_1$-$C_4$-Alkylthio (welches gegebenenfalls durch Cyano, Fluor und/oder Chlor substituiert ist), $C_1$-$C_4$-Alkyl, $C_1$-$C_4$-Alkoxy-carbonyl, Aminocarbonyl, $C_1$-$C_4$-Alkyl-amino-carbonyl, Di-($C_1$-$C_4$-alkyl)-amino-carbonyl, Amino, $C_1$-$C_4$-Alkylamino oder Di-($C_1$-$C_4$-alkyl)-amino steht,
$R^2$ für Wasserstoff, $C_1$-$C_4$-Alkyl, $C_2$-$C_4$-Alkenyl oder $C_2$-$C_4$-Alkinyl steht,
$R^3$ für eine gegebenenfalls durch Fluor, Chlor, Brom, Iod, Cyano, Nitro, $C_1$-$C_4$-Alkyl (welches gegebenenfalls durch Fluor und/oder Chlor substituiert ist), $C_1$-$C_4$-Alkoxy oder $C_1$-$C_2$-Alkylendioxy (welche gegebenenfalls durch Fluor und/oder Chlor substituiert sind), $C_1$-$C_4$-Alkylthio, $C_1$-$C_4$-Alkyl-sulfinyl oder $C_1$-$C_4$-Alkylsulfonyl (welche gegebenenfalls durch Fluor und/oder Chlor substituiert sind), Di-($C_1$-$C_2$-alkyl)-amino und/oder durch $C_1$-$C_4$-Alkoxy-carbonyl substituierte aromatische bzw. heteroaromatische Gruppierung aus der Reihe Phenyl, Naphthyl, Pyridyl, Chinolinyl, Isochinolinyl, Pyrimidinyl, Furyl, Thienyl, Pyrrolyl, Pyrazolyl oder Imidazolyl steht,
$R^4$ für Wasserstoff, $C_1$-$C_4$-Alkyl, $C_2$-$C_4$-Alkenyl oder $C_2$-$C_4$-Alkinyl steht und
$R^5$ für Wasserstoff oder $C_1$-$C_4$-Alkyl steht.

Die Erfindung betrifft insbesondere Verbindungen der Formel (I), in welcher
$A^1$ für gegebenenfalls verzweigtes und gegebenenfalls durch Phenyl substituiertes Alkandiyl ("Alkylen") mit 1 bis 3 Kohlenstoffatomen steht,
$A^2$ für gegebenenfalls verzweigtes Alkandiyl ("Alkylen") mit 1 bis 3 Kohlenstoffatomen steht,
Q für Sauerstoff, Schwefel, NH oder N-$CH_3$ steht,
$R^1$ für Cyanamino, Azido, Fluor, Chlor, Brom, Methoxy, Ethoxy, Methylthio, Ethylthio, Difluormethylthio, Cyanomethylthio, Methyl, Amino, Methylamino oder Dimethylamino steht,
$R^2$ für Wasserstoff oder Methyl steht,
$R^3$ für gegebenenfalls durch Fluor, Chlor, Brom, Cyano, Nitro, Methy, Ethyl, Methoxy und/oder Ethoxy substituiertes Phenyl oder Naphthyl, für gegebenenfalls durch Fluor, Chlor, Brom, Cyano, Nitro, Methyl, Ethyl, Methoxy und/oder Ethoxy substituiertes Pyridyl oder für gegebenenfalls durch Fluor, Chlor, Brom, Methyl und/ oder Ethyl substituiertes Furyl steht,
$R^4$ für Wasserstoff oder Methyl steht und

$R^5$ für $C_1$-$C_3$-Alkyl steht.

Ganz besonders bevorzugt werden diejenigen Verbindungen der Formel (I), in welcher
$A^1$ für Ethan-1,1-diyl ("Ethyliden") steht, wobei die an das C-Atom in 1-Position des Ethan-1,1-diyl gebundenen Gruppierungen in einer S-Konfiguration angeordnet sind,
$A^2$ für Ethan-1,2-diyl ("Ethylen") oder Propan-1,3-diyl ("Trimethylen") steht,
Q für Sauerstoff steht,
$R^1$ für Chlor, Methoxy oder Methylthio steht,
$R^2$ für Wasserstoff steht,
$R^3$ für Phenyl steht,
$R^4$ für Wasserstoff steht und
$R^5$ für Methyl oder Ethyl steht.

Verwendet man beispielsweise 2,4-Dichlor-6-benzylamino-s-triazin und 3-Methoxy-propylamin als Ausgangsstoffe, so kann der Reaktionsablauf beim erfindungsgemäßen Verfahren (a) durch das folgende Formelschema skizziert werden:

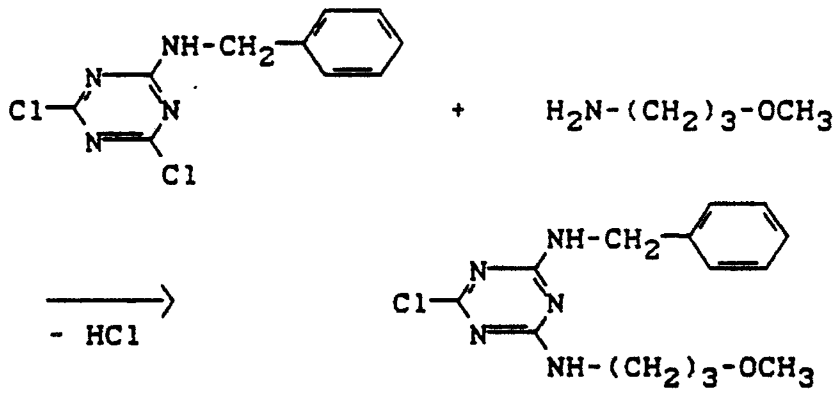


Verwendet man beispielsweise 2-Chlor-4-methoxy-6-(2-ethoxy-ethylamino)-s-triazin und 2-Phenylethylamin als Ausgangsstoffe, so kann der Reaktionsablauf beim erfindungsgemäßen Verfahren (b) durch das folgende Formelschema skizziert werden:

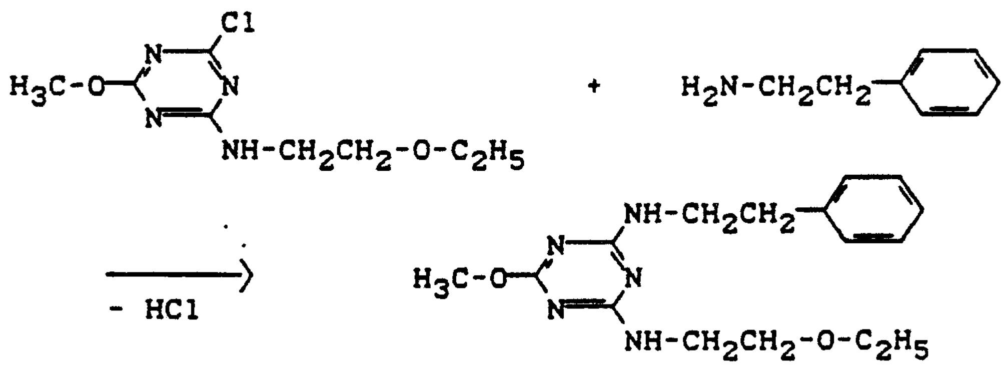


Verwendet man beispielsweise 2-Chlor-4-(2-methoxy-ethylamino)-6-(2-chlor-pyridin-5-yl-methylamino)-s-triazin und Natrium-methanthiolat als Ausgangsstoffe, so kann der Reaktionsablauf beim erfindungsgemäßen Verfahren (c) durch das folgende Formelschema skizziert werden:

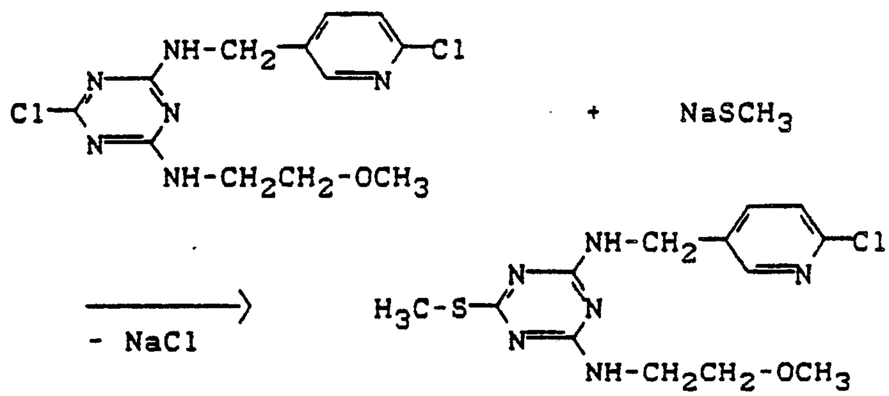


Verwendet man beispielsweise 2-Mercapto-4-(2-methoxyethylamino)-6-(2-phenyl-ethylamino)-s-triazin und Chloracetonitril als Ausgangsstoffe, so kann der Reaktionsablauf beim erfindungsgemäßen Verfahren (d) durch das folgende Fomelschema skizziert werden:

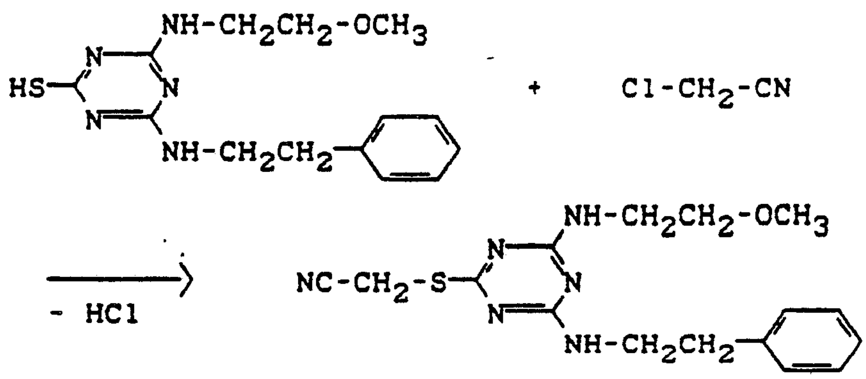


Die beim erfindungsgemäßen Verfahren (a) als Ausgangsstoffe zu verwendenden halogenierten Triazine sind durch die Formel (II) allgemein definiert. In Formel (II) haben $A^1$, $R^1$, $R^2$ und $R^3$ vorzugsweise bzw. insbesondere bzw. ganz besonders bevorzugt diejenigen Bedeutungen, die bereits oben im Rahmen der Beschreibung der erfindungsgemäßen Verbindungen der Formel (I) als bevorzugt bzw. als insbesondere bevorzugt bzw. als ganz besonders bevorzugt für $A^1$, $R^1$, $R^2$ bzw. $R^3$ angegeben wurden und X steht vorzugsweise für Fluor, Chlor oder Brom, insbesondere für Chlor.

Beispiele für die Ausgangsstoffe der Formel (II) sind in der nachstehenden Tabelle 1 aufgeführt.

Tabelle 1: Beispiele für die Ausgangsstoffe der Formel (II)

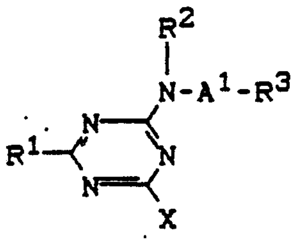

(II)

| $A^1$ | $R^1$ | $R^2$ | $R^3$ | X |
|---|---|---|---|---|
| $CH_2$ | Cl | H | 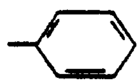 | Cl |
| $CHCH_3$ | Cl | H | 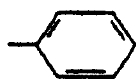 | Cl |
| $CH_2$ | Cl | $CH_3$ | 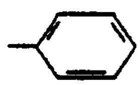 | Cl |
| $CH_2$ | Cl | H | 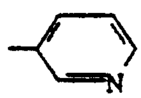 | Cl |
| $CH_2$ | Cl | H | 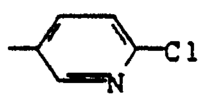 | Cl |
| $CHCH_3$ | Cl | $CH_3$ | 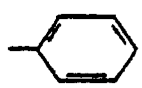 | Cl |
| $CHCH_3$ | $OCH_3$ | H | 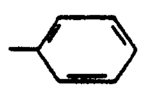 | Cl |

Tabelle 1 - Fortsetzung

| $A^1$ | $R^1$ | $R^2$ | $R^3$ | X |
|---|---|---|---|---|
| $CHCH_3$ | $SCH_3$ | H | 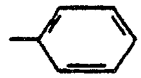 | Cl |
| $CHCH_3$ | Cl | H | 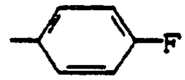 | Cl |
| $CHCH_3$ | Cl | H | 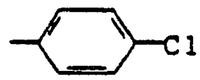 | Cl |
| $CH_2CH_2$ | Cl | H | 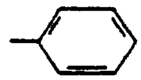 | Cl |
| $CH_2$ | Cl | H | 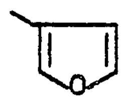 | Cl |
| $CH_2$ | Cl | H | 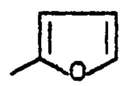 | Cl |
| $CH_2$ | Cl | H | 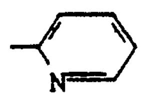 | Cl |
| $CHCH_3$ | NHCN | H | 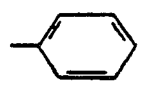 | Cl |
| $CH_2$ | Cl | H | 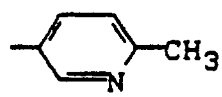 | Cl |
| $CH_2$ | Cl | H | 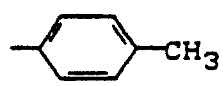 | Cl |
| $CH_2$ | Cl | H | 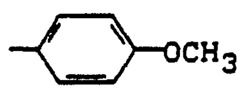 | Cl |

Tabelle 1 - Fortsetzung

| $A^1$ | $R^1$ | $R^2$ | $R^3$ | X |
|---|---|---|---|---|
| $CH_2$ | Cl | H | 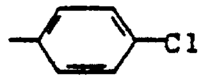 | Cl |
| $CHCH_3$ | $CH_3$ | H | 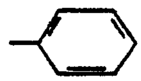 | Cl |

Die halogenierten Triazine der Formel (II) sind bekannt und/oder können nach an sich bekannten Verfahren hergestellt werden (vgl. Zh. Obshch. Khim. 42 (1972), 2280 - 2284, zit. in Chem. Abstracts 78 (1973), 72558w).

Man erhält die Verbindungen der Formel (II), wenn man die entsprechenden dihalogenierten bzw. trihalogenierten Triazine, wie z. B. 2,4,6-Trichlor-s-triazin ("Cyanurchlorid") oder 2,4-Dichlor-6-methyl-s-triazin oder 2,4-Dichlor-6-cyanamino-s-triazin, mit geeigneten Aminen, wie z. B. Benzylamin, 2-Phenylethylamin, R- oder S-oder R/S-1-Phenyl-ethylamin oder 3-Pyridyl-methylamin, gegebenenfalls in Gegenwart eines Verdünnungsmittels, wie z. B. Tetrahydrofuran, und gegebenenfalls in Gegenwart eines Säureakzeptors, wie z. B. Triethylamin oder Ethyl-diisopropylamin, bei Temperaturen zwischen +30 °C und -80 °C umsetzt.

Die bei Verfahren (a) weiter als Ausgangsstoffe zu verwendenden Aminoverbindungen sind durch die Formel (III) allgemein definiert.

In Formel (III) haben $A^2$, Q, $R^4$ und $R^5$ vorzugsweise bzw. insbesondere bzw. ganz besonders bevorzugt diejenigen Bedeutungen, die bereits oben im Rahmen der Beschreibung der erfindungsgmäßen Verbindungen der Formel (I) als bevorzugt bzw. als insbesondere bevorzugt bzw. als ganz besonders bevorzugt für $A^2$, Q, $R^4$ bzw. $R^5$ angegeben wurden.

Beispiele für die Ausgangsstoffe der Formel (III) sind in der nachstehenden Tabelle 2 aufgeführt.

Tabelle 2: Beispiele für die Ausgangsstoffe der Formel (III)

$$\begin{array}{l} H\text{-}N\text{-}A^2\text{-}Q\text{-}R^5 \\ \quad\; | \\ \quad R^4 \end{array} \qquad (III)$$

| $A^2$ | Q | $R^4$ | $R^5$ |
|---|---|---|---|
| $CH_2$ | O | H | $CH_3$ |
| $CH_2CH_2$ | O | H | $CH_3$ |
| $CH_2CH_2$ | S | H | $CH_3$ |
| $CH_2CH_2$ | NH | H | $CH_3$ |
| $CH_2CH_2$ | $NCH_3$ | H | $CH_3$ |
| $CH_2CH_2$ | O | $CH_3$ | $CH_3$ |
| $CH_2CH_2$ | S | H | $C_2H_5$ |
| $CH_2CH_2$ | O | H | $C_2H_5$ |
| $(CH_2)_3$ | O | H | $CH_3$ |
| $(CH_2)_3$ | O | H | $C_2H_5$ |
| $CH_2CH_2$ | O | $CH_3$ | $C_2H_5$ |
| $\begin{array}{l}CHCH_2\\ \vert\\ CH_3\end{array}$ | O | H | $CH_3$ |

Die Aminoverbindungen der Formel (III) sind bekannte Synthesechemikalien.

Die beim erfindungsgemäßen Verfahren (b) als Ausgangsstoffe zu verwendenden halogenierten Triazine sind durch die Formel (IV) allgemein definiert. In Formel (IV) haben $A^2$, Q, $R^1$, $R^4$ und $R^5$ vorzugsweise bzw. insbesondere bzw. ganz besonders bevorzugt diejenigen Bedeutungen, die bereits im Rahmen der Beschreibung der erfindungsgemäßen Verbindungen der Formel (I) als bevorzugt bzw. als insbesondere bevorzugt bzw. als ganz besonders bevorzugt für $A^2$, Q, $R^1$, $R^4$ bzw. $R^5$ angegeben wurden und Y steht vorzugsweise für Fluor, Chlor oder Brom, insbesondere für Chlor.

Beispiele für die Ausgangsstoffe der Formel (IV) sind in der nachstehenden Tabelle 3 aufgeführt.

Tabelle 3: Beispiele für die Ausgangsstoffe der Formel (IV)

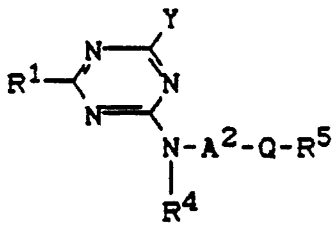

(IV)

Tabelle 3

| $A^2$ | Q | $R^1$ | $R^4$ | $R^5$ | Y |
|---|---|---|---|---|---|
| $CH_2$ | O | Cl | H | $CH_3$ | Cl |
| $CH_2CH_2$ | O | Cl | H | $CH_3$ | Cl |
| $CH_2CH_2$ | S | Cl | H | $CH_3$ | Cl |
| $CH_2CH_2$ | NH | Cl | H | $CH_3$ | Cl |
| $CH_2CH_2$ | $NCH_3$ | Cl | H | $CH_3$ | Cl |
| $CH_2CH_2$ | O | Cl | $CH_3$ | $CH_3$ | Cl |
| $CH_2CH_2$ | O | Cl | H | $C_2H_5$ | Cl |
| $(CH_2)_3$ | O | Cl | H | $CH_3$ | Cl |
| $(CH_2)_3$ | O | Cl | H | $C_2H_5$ | Cl |
| $CH_2$ | O | Cl | H | $C_2H_5$ | Cl |
| $CH_2CH_2$ | O | Cl | H | $C_2H_5$ | Cl |
| $CH_2CH_2$ | O | $OCH_3$ | H | $CH_3$ | Cl |
| $CH_2CH_2$ | O | $SCH_3$ | H | $CH_3$ | Cl |
| $CH_2CH_2$ | O | $CH_3$ | H | $CH_3$ | Cl |
| $CH_2CH_2$ | O | NHCN | H | $CH_3$ | Cl |
| $CH_2CH_2$ | O | Cl | $CH_3$ | $C_2H_5$ | Cl |
| $CH(CH_3)-CH_2$ | O | Cl | H | $CH_3$ | Cl |

Die halogenierten Triazine der Formel (IV) sind bekannt und/oder können nach an sich bekannten Verfahren hergestellt werden (vgl. CH-PS 605 853, zit. in Chem. Abstracts 90 (1979), 54988e; DD-PS 51

646, zit. in Chem. Abstracts 66 (1967), 105005w).

Man erhält die Verbindungen der Formel (IV), wenn man entsprechende dihalogenierte bzw. trihalogenierte Triazine, wie z. B. 2,4,6-Trichlor-s-triazin ("Cyanurchlorid") oder 2,4-Dichlor-6-methyl-s-triazin oder 2,4-Dichlor-6-cyanamino-s-triazin, mit geeigneten Aminen, wie z. B. 2-Methoxyethylamin, 2-Ethoxy-ethylamin, 2-Methylthioethylamin, 2-Methylamino-ethylamin oder 3-Methoxy-propylamin, gegebenenfalls in Gegenwart eines Verdünnungsmittels, wie z. B. Tetrahydrofuran, und gegebenenfalls in Gegenwart eines Säureakzeptors, wie z. B. Triethylamin oder Ethyl-diisopropylamin, bei Temperaturen zwischen +30 °C und -80 °C umsetzt.

Die bei Verfahren (b) weiter als Ausgangsstoffe zu verwendenden Aminoverbindungen sind durch die Formel (V) allgemein definiert.

In Formel (V) haben $A^1$, $R^2$ und $R^3$ vorzugsweise bzw. insbesondere bzw. ganz besonders bevorzugt diejenigen Bedeutungen, die bereits oben im Rahmen der Beschreibung der erfindungsgemäßen Verbindungen der Formel (I) als bevorzugt bzw. als insbesondere bevorzugt bzw. als ganz besonders bevorzugt für $A^1$, $R^2$ und $R^3$ angegeben wurden.

Beispiele für die Ausgangsstoffe der Formel (V) sind in der nachstehenden Tabelle 4 aufgeführt.

<u>Tabelle 4:</u> Beispiele für die Ausgangsstoffe der Formel (V)

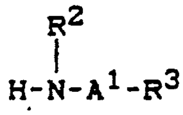 (V)

| $A^1$ | $R^2$ | $R^3$ |
| --- | --- | --- |
| $CH_2$ | H | 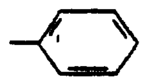 |
| $CHCH_3$ | H | 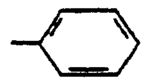 |
| $CH_2$ | $CH_3$ | 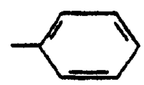 |
| $CH_2$ | H | 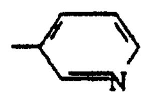 |
| $CH_2$ | H | 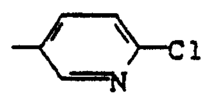 |
| $CHCH_3$ | H | 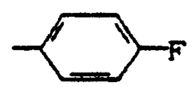 |
| $CHCH_3$ | H | 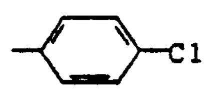 |
| $CH_2CH_2$ | H | 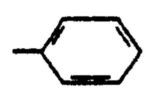 |
| $CH_2$ | H | 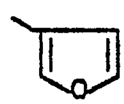 |

Tabelle 4 - Fortsetzung

| $A^1$ | $R^2$ | $R^3$ |
|---|---|---|
| $CH_2$ | H | 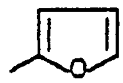 |
| $CH_2$ | H | 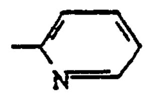 |
| $CH_2$ | H | 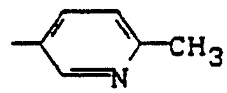 |
| $CH_2$ | H | 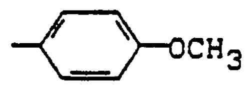 |
| $CH_2$ | H | 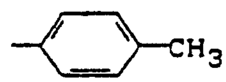 |
| $CH_2$ | H | 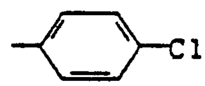 |

Die Aminoverbindungen der Formel (V) sind bekannt und/oder können nach an sich bekannten Verfahren hergestellt werden (vgl. EP-A 192 060).

Die beim erfindungsgemäßen Verfahren (c) als Ausgangsstoffe zu verwendenden halogenierten Triazine sind durch die Formel (VI) allgemein definiert. In Formel (VI) haben $A^1$, $A^2$, Q, $R^2$, $R^3$, $R^4$ und $R^5$ vorzugsweise bzw. insbesondere bzw. ganz besonders bevorzugt diejenigen Bedeutungen, die bereits im Rahmen der Beschreibung der erfindungsgemäßen Verbindungen der Formel (I) als bevorzugt bzw. als insbesondere bevorzugt bzw. als ganz besonders bevorzugt für $A^1$, $A^2$, Q, $R^2$, $R^3$, $R^4$ und $R^5$ angegeben wurden und Z steht vorzugsweise für Fluor, Chlor oder Brom, insbesondere für Chlor.

Beispiele für die Ausgangsstoffe der Formel (VI) sind in der nachstehenden Tabelle 5 aufgeführt.

Tabelle 5: Beispiele für die Ausgangsstoffe der Formel (VI)

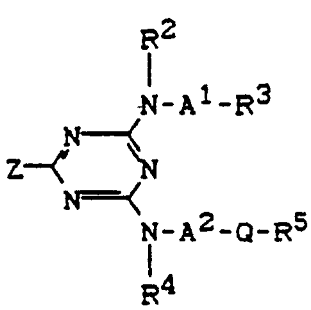

(VI)

Tabelle 5

| $A^1$ | $A^2$ | Q | $R^2$ | $R^3$ | $R^4$ | $R^5$ | Z |
|---|---|---|---|---|---|---|---|
| $CH_2$ | $CH_2CH_2$ | O | H | 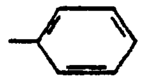 | H | $CH_3$ | Cl |
| $CH_2CH_2$ | $CH_2CH_2$ | O | H | 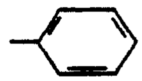 | H | $CH_3$ | Cl |
| $CHCH_3$ | $CH_2CH_2$ | O | H | 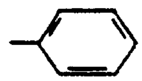 | H | $CH_3$ | Cl |
| $CHCH_3$ | $CH_2CH_2$ | O | H | 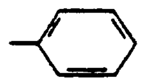 | H | $C_2H_5$ | Cl |
| $CH_2$ | $CH_2CH_2$ | O | $CH_3$ | 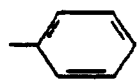 | H | $CH_3$ | Cl |
| $CHCH_3$ | $(CH_2)_3$ | O | H | 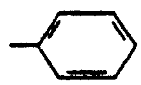 | H | $CH_3$ | Cl |
| $CH_2$ | $CH_2CH_2$ | O | H | 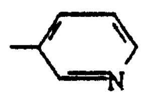 | H | $CH_3$ | Cl |
| $CH_2$ | $CH_2CH_2$ | O | H | 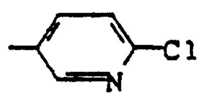 | H | $CH_3$ | Cl |
| $CHCH_3$ | $CH_2CH_2$ | O | H | 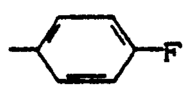 | H | $CH_3$ | Cl |
| $CHCH_3$ | $CH_2CH_2$ | O | H | 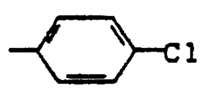 | H | $CH_3$ | Cl |
| $CH_2$ | $CH_2CH_2$ | O | H | 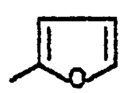 | H | $CH_3$ | Cl |

Tabelle 5 - Fortsetzung

| $A^1$ | $A^2$ | Q | $R^2$ | $R^3$ | $R^4$ | $R^5$ | Z |
|---|---|---|---|---|---|---|---|
| $CH_2$ | $CH_2CH_2$ | O | H | 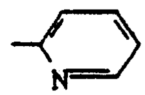 | H | $C_2H_5$ | Cl |
| $CH_2$ | $CH_2CH_2$ | O | H | 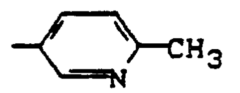 | H | $CH_3$ | Cl |
| $CH_2$ | $CH_2CH_2$ | O | H | 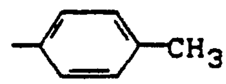 | H | $C_2H_5$ | Cl |
| $CH_2$ | $CH_2CH_2$ | O | H | 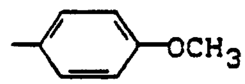 | H | $CH_3$ | Cl |
| $CH_2$ | $CH_2CH_2$ | O | H | 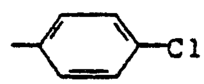 | H | $CH_3$ | Cl |
| $CHCH_3$ | $CH_2CH_2$ | O | H | 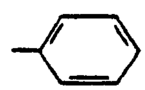 | $CH_3$ | $CH_3$ | Cl |
| $CHCH_3$ | $CH_2$ | O | H | 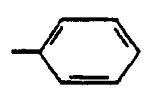 | H | $CH_3$ | Cl |
| $CH_2$ | $\underset{CH_3}{\underset{\vert}{C}}HCH_2$ | O | H | 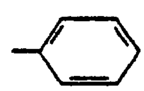 | H | $CH_3$ | Cl |
| $CHC_2H_5$ | $CH_2CH_2$ | O | H | 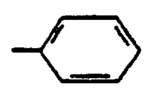 | H | $C_2H_5$ | Cl |

Die Ausgangsstoffe der Formel (VI) sind auch erfindungsgemäße Wirkstoffe, welche zum Definitionsbereich der Formel (I) gehören. Sie können nach den erfindungsgemäßen Verfahren (a) und (b) hergestellt werden.

Die bei Verfahren (c) einzusetzenden Alkylamine, Dialkylamine, Alkanole bzw. Alkanthiole, vorzugsweise jeweils mit bis zu 4 Kohlenstoffatomen, insbesondere mit 1 oder 2 Kohlenstoffatomen, wie z. B. Methylamin, Ethylamin, Dimethylamin, Methanol, Ethanol, Methanthiol und Ethanthiol sowie deren Natriumsalze oder Kaliumsalze, sind bekannte Synthesechemikalien.

Die beim erfindungsgemäßen Verfahren (d) als Ausgangsstoffe zu verwendenden Mercaptotriazine sind durch die Formel (VII) allgemein definiert. In Formel (VII) haben $A^1$, $A^2$, Q, $R^2$, $R^3$, $R^4$ und $R^5$ vorzugsweise bzw. insbesondere bzw. ganz besonders bevorzugt diejenigen Bedeutungen, die bereits im Rahmen der Beschreibung der erfindungsgemäßen Verbindungen der Formel (I) als bevorzugt bzw. als insbesondere bevorzugt bzw. als ganz besonders bevorzugt für $A^1$, $A^2$, Q, $R^2$, $R^3$, $R^4$ und $R^5$ angegeben wurden.

Beispiele für die Ausgangsstoffe der Formel (VII) sind in der nachstehenden Tabelle 6 aufgeführt.

<u>Tabelle 6</u>: Beispiele für die Ausgangsstoffe der Formel (VII)

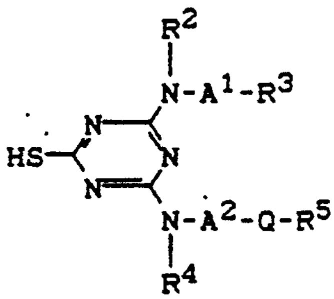

(VII)

| $A^1$ | $A^2$ | Q | $R^2$ | $R^3$ | $R^4$ | $R^5$ |
|---|---|---|---|---|---|---|
| $CH_2$ | $CH_2CH_2$ | O | H | 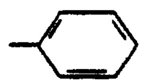 | H | $CH_3$ |
| $CH_2CH_2$ | $CH_2CH_2$ | O | H | 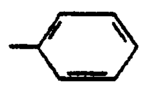 | H | $CH_3$ |
| $CHCH_3$ | $CH_2CH_2$ | O | H | 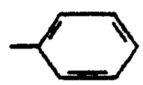 | H | $CH_3$ |
| $CHCH_3$ | $CH_2CH_2$ | O | H | 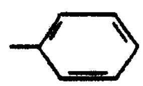 | H | $C_2H_5$ |
| $CH_2$ | $CH_2CH_2$ | O | $CH_3$ | 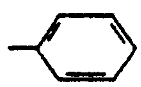 | H | $CH_3$ |
| $CHCH_3$ | $(CH_2)_3$ | O | H | 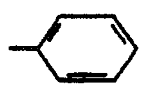 | H | $CH_3$ |
| $CHCH_3$ | $CH_2CH_2$ | O | H | 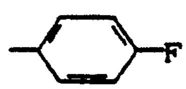 | H | $C_2H_5$ |
| $CHCH_3$ | $CH_2CH_2$ | O | H | 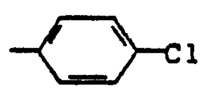 | H | $C_2H_5$ |

Tabelle 6 - Fortsetzung

| $A^1$ | $A^2$ | Q | $R^2$ | $R^3$ | $R^4$ | $R^5$ |
|---|---|---|---|---|---|---|
| $CHCH_3$ | $CH_2CH_2$ | O | H | 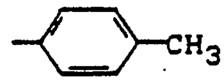 | H | $C_2H_5$ |
| $CHCH_3$ | $CH_2CH_2$ | O | H | 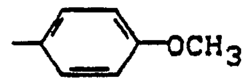 | H | $CH_3$ |
| $CH_2$ | $CH_2CH_2$ | O | H | 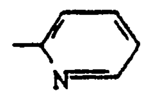 | H | $C_2H_5$ |
| $CH_2$ | $CH_2CH_2$ | O | H | 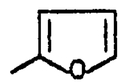 | H | $C_2H_5$ |

Die Mercaptotriazine der Formel (VII) sind noch nicht aus der Literatur bekannt.

Man erhält die neuen Verbindungen der Formel (VII), wenn man halogenierte Triazine de Formel (VI) - oben - zunächst mit Thioharnstoff, vorzugsweise in Gegenwart eines Verdünnungsmittels, wie z. B. Dioxan, bei Temperaturen zwischen 0 °C und 150 °C umsetzt und dann mit einer wäßrigen Alkalihydroxidlösung, vorzugsweise 2N-Natronlauge, bei Temperaturen zwischen 50 °C und 120 °C umsetzt und nach üblichen Methoden aufarbeitet.

Die bei Verfahren (d) einzusetzenden Halogenalkylverbindungen, vorzugsweise durch Fluor oder Cyano substituierte Chlor- oder Brom-alkane mit 1 bis 4 Kohlenstoffatomen, insbesondere mit 1 oder 2 Kohlenstoffatomen, wie z. B. Chlordifluormethan, Chloracetonitril, Bromacetonitril oder Brompropionitril, sind bekannte Synthesechemikalien.

Das erfindungsgemäße Verfahren (a) zur Herstellung der neuen substituierten Triazine der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Gly koldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Ethyl-diisopropylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO). Auch die Ausgangsstoffe der Formel (III) können als Säureakzeptoren dienen.

Die Reaktionstemperaturen können bei dem erfindungsgemäßen Verfahren (a) in einem größeren Bereich variiert werden. Im allgemeinen arbeitet man bei Temperaturen zwischen -30 °C und +50 °C, vorzugsweise bei Temperaturen zwischen -20 °C und +40 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die Ausgangsstoffe der Formeln (II) und (III) im allgemeinen in angenähert äquimolaren Mengen eingesetzt. Bei Verzicht auf gesonderte Säureakzeptoren verwendet man im allgemeinen etwa 2 Mol Aminoverbindung der Formel (III) je Mol Triazin der Formel (II).

Die Reaktionskomponenten werden im allgemeinen bei Raumtemperatur oder unter Kühlen zusammengegeben und das Reaktionsgemisch wird bis zum Ende der Umsetzung gerührt. Die Aufarbeitung kann nach üblichen Methoden durchgeführt werden.

Das erfindungsgemäße Verfahren (b) zur Herstellung der neuen substituierten Triazine der Formel (I) wird vorzugsweise unter Verwendung von Verdünnungsmitteln durchgeführt, wie sie oben für Verfahren (a) angegeben sind.

Verfahren (b) wird vorzugsweise unter Verwendung von Säureakzeptoren durchgeführt, wie sie oben für Verfahren (a) angegeben sind. Auch die Ausgangsstoffe der Formel (V) können als Säureakzeptoren dienen.

Die Reaktionstemperaturen können bei dem erfindungsgemäßen Verfahren (b) in einem größeren Bereich variiert werden. Im allgemeinen arbeitet man bei Temperaturen zwischen -30 °C und +50 °C, vorzugsweise bei Temperaturen zwischen -20 °C und +40 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die Ausgansstoffe der Formeln (IV) und (V) im allgemeinen in angenähert äquimolaren Mengen eingesetzt. Bei Verzicht auf gesonderte Säureakzeptoren verwendet man im allgemeinen etwa 2 Mol Aminoverbindung der Formel (V) je Mol Triazin der Formel (IV).

Die Reaktionskomponenten werden im allgemeinen bei Raumtemperatur oder unter Kühlen zusammengegeben und das Reaktionsgemisch wird bis zum Ende der Umsetzung gerührt. Die Aufarbeitung kann nach üblichen Methoden durchgeführt werden.

Das erfindungsgemäße Verfahren (c) zur Herstellung der neuen substituierten Triazine der Formel (I) wird vorzugsweise unter Verwendung von Verdünnungsmitteln durchgeführt, wie sie oben für Verfahren (a) angegeben sind.

Verfahren (c) kann unter Verwendung von Säureakzeptoren durchgeführt werden, wie sie oben für Verfahren (a) angegeben sind. Vorzugsweise werden jedoch im Falle der Umsetzung zu Alkoxy- bzw. Alkylthioverbindungen unter Verzicht auf gesonderte Säureakzeptoren die Natriumsalze oder Kaliumsalze der bei Verfahren (c) benötigten Alkanole bzw. Alkanthiole eingesetzt.

Die Reaktionstemperaturen können bei dem erfindungsgemäßen Verfahren (c) in einem größeren Bereich variiert werden. Im allgemeinen arbeitet man bei Temperaturen zwischen -20 °C und +80 °C, vorzugsweise bei Temperaturen zwischen 0 °C und 50 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) setzt man im allgemeinen je Mol Ausgangsverbindung der Formel (VI) zwischen 1 und 2 Mol, vorzugsweise zwischen 1,0 und 1,2 Mol, eines Alkanols bzw. Alkanthiols bzw. von dessen Alkalimetallsalzen ein.

Die Reaktionskomponenten werden im allgemeinen bei Raumtemperatur oder unter Kühlen zusammengegeben und das Reaktionsgemisch wird bis zum Ende der Umsetzung gerührt. Die Aufarbeitung kann nach üblichen Methoden durchgeführt werden.

Das erfindungsgemäße Verfahren (d) zur Herstellung der neuen substituierten Triazine der Formel (I) wird vorzugsweise unter Verwendung von Verdünnungsmitteln durch geführt, wie sie oben für Verfahren (a) angegeben sind. Es können jedoch auch Wasser und Alkohole, wie z. B. Methanol, Ethanol, Propanol, Isopropanol, Butanol, Isobutanol, sec-Butanol oder tert-Butanol als Verdünnungsmittel verwendet werden.

Verfahren (d) wird vorzugsweise unter Verwendung von Säureakzeptoren durchgeführt, wie sie oben für Verfahren (a) angegeben sind.

Die Reaktionstemperaturen können bei dem erfindungsgemäßen Verfahren (d) in einem größeren Bereich variiert werden. Im allgemeinen arbeitet man bei Temperaturen zwischen 0 °C und 100 °C, vorzugsweise bei Temperaturen zwischen 10 °C und 80 °C.

Das erfindungsgemäße Verfahren (d) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (d) setzt man je Mol Ausgangsverbindung der Formel (VII) im allgemeinen zwischen 1 und 10 Mol, vorzugsweise zwischen 1 und 3 Mol, Halogenalkylverbindung der Formel (VIII) ein.

Vorzugsweise wird das Mercaptotriazin der Formel (VII) mit einem Verdünnungsmittel und einem Säureakzeptor vorgelegt und die Halogenalkylverbindung der Formel (VIII) wird langsam eindosiert. Die Aufarbeitung kann nach üblichen Methoden durchgeführt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle

Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u>

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dilotyle Kulturen der Gattungen:</u>

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u>

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u>

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich zur selektiven Bekämpfung monokotyler und dikotyler Unkräuter in monokotylen Kulturen, insbesondere im Nachauflaufverfahren.

Sie sind bei guter Verträglichkeit für Mais, Gerste und Weizen deutlich wirksamer gegen monokotyle und dikotyle Unkräuter als das bekannte Atrazin.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorgansichen und organischen Mehlen sowie Granulate aus organischem Material wie Sägemehl, Kokosnußschalen, Maiskolben und Tabakstengeln; als Emulgier- und/oder schaumerzeugende Mittel kommen in Frage:

z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wo bei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage; ferner auch 2,4-Dichlorphenoxyessigsäure; 2,4-Dichlorphenoxypropionsäure; 4-(2,4-Dichlorphenoxy)-buttersäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Chlor-N-(2,6-dimethylphenyl)-N-[(1H)-pyrazol-1-yl-methyl]-acetamid; 2-Ethyl-6-methyl-N-(1-methyl-2-methoxyethyl)-chloracetanilid; N-(1-Ethylpropyl)-3,4-dimethyl-2,6-dinitroanilin; 3,5-Dibrom-4-hydroxy-benzonitril; 3,5-Diiod-4-hydroxybenzonitril; N-Methyl-2-(1,3-benzthiazol-2-yloxy)-acetanilid; 3-[[[[(4-Methoxy-6-methyl-1,3,5-triazin-2-yl)-amino]-carbonyl]-amino]-sulfonyl]-thiophen-2-carbonsäure-methylester; 2-Chlor-4-ethylamino-6-isopropylamino-1,3,5-triazin; 2-Chlor-4-ethylamino-6-(3-cyanopropylamino)-1,3,5-triazin; 3-Isopropyl-2,1,3-benzothiadiazin-4-on-2,2-dioxid und O-(6-Chlor-3-phenyl-pyridazin-4-yl)-S-octyl-thiocarbonat. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

<u>Herstellungsbeispiele</u>

Beispiel 1

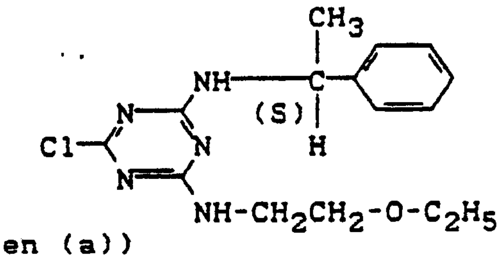

(Verfahren (a))

Eine Lösung von 3,56 g (0,04 Mol) 2-Ethoxy-ethylamin in 20 ml Tetrahydrofuran wird unter Rühren zu einer auf -10 °C gekühlten Lösung von 5,4 g (0,02 Mol) (S)-2,4-Dichlor-6-(1-phenyl-ethylamino)-s-triazin in 50 ml Tetrahydrofuran innerhalb von etwa 20 Minuten tropfenweise gegeben. Das Reaktionsgemisch wird dann bei -10 °C 30 Minuten gerührt und anschließend wird das kristallin angefallene Produkt durch Absaugen isoliert.

Man erhält 6,4 g (99 % der Theorie) (S)-2-Chlor-4-(2-ethoxy-ethylamino)-6-(1-phenyl-ethylamino)-s-triazin vom Schmelzpunkt 103 °C.

Beispiel 2

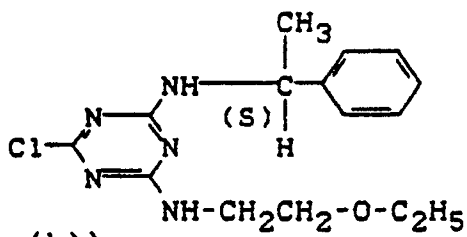

(Verfahren (b))

Eine Lösung von 10,9 g (0,09 Mol) (S)-1-Phenyl-ethylamin in 100 ml Tetrahydrofuran und eine Lösung von 9,1 g (0,09 Mol) Triethylamin in 50 ml Tetrahydrofuran werden zu einer auf -10 °C gekühlten Lösung von 21,3 g (0,09 Mol) 2,4-Dichlor-6-(2-ethoxy-ethylamino)-s-triazin in 100 ml Tetrahydrofuran unter Rühren tropfenweise gegeben. Nach Entfernen der Kühlung wird das Reaktionsgemisch bei -10 °C bis +20 °C etwa 60 Minuten gerührt, dann im Wasserstrahlvakuum eingeengt, in 50 ml Methylenchlorid aufgenommen, erneut eingeengt und der Rückstand mit Petrolether verrührt.

Man erhält nach Absaugen 17,4 g (60 % der Theorie) (S)-2-Chlor-4-(2-ethoxy-ethylamino)-6-(1-phenyl-ethylamino)-s-triazin vom Schmelzpunkt 103 °C.

Beispiel 3

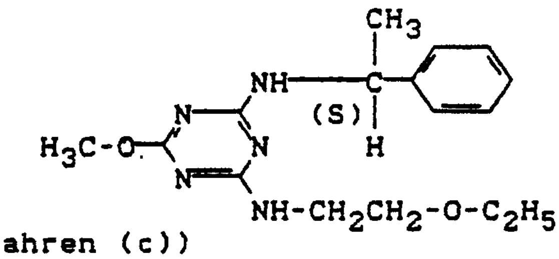

(Verfahren (c))

Eine Mischung aus 1,6 g (0,005 Mol) (S)-2-Chlor-4-(2-ethoxy-ethylamino)-6-(1-phenyl-ethylamino)-s-triazin, 0,27 g (0,005 Mol) Natrium-methanolat und 20 ml Tetrahydrofuran wird etwa 20 Stunden bei 20 °C gerührt. Man engt ein, nimmt den Rückstand in Wasser auf, extrahiert mit Methylenchlorid, trocknet die Extraktionslösung mit Natriumsulfat, filtriert und destilliert vom Filtrat das Lösungsmittel im Wasserstrahlvakuum ab.

Man erhält 1,4 g (80 % der Theorie) (S)-2-Methoxy-4-(2-ethoxy-ethylamino)-6-(1-phenyl-ethylamino)-s-triazin als öligen Rückstand vom Brechungsindex $n_D^{22}$ = 1,5610.

Analog Beispiel 1, 2 und 3 sowie entsprechend der allgemeinen Beschreibung der erfindungsgemäßen Verfahren (a), (b), (c) und (d) können die in der nachstehenden Tabelle 7 aufgeführten Verbindungen der Formel (I) hergestellt werden.

**Tabelle 7: Beispiele für die Verbindungen der Formel (I)**

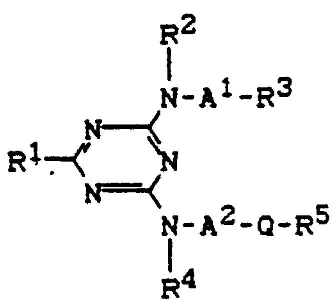

(I)

Tabelle 7

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 4 | (S)-$CHCH_3$ | $CH_2CH_2$ | O | Cl | H | 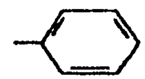 | H | $CH_3$ | 115 °C |
| 5 | (S)-$CHCH_3$ | $(CH_2)_3$ | O | Cl | H | 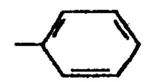 | H | $CH_3$ | 128 °C |
| 6 | (S)-$CHCH_3$ | $CH_2CH_2$ | O | $SCH_3$ | H | 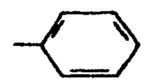 | H | $CH_3$ | |
| 7 | (S)-$CHCH_3$ | $CH_2CH_2$ | O | $SCH_3$ | H | 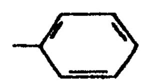 | H | $C_2H_5$ | (amorph) |
| 8 | (S)-$CHCH_3$ | $CH_2CH_2$ | O | $OCH_3$ | H | 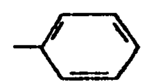 | H | $CH_3$ | |
| 9 | (R)-$CHCH_3$ | $CH_2CH_2$ | O | Cl | H | 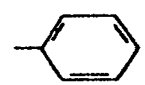 | H | $C_2H_5$ | 102 °C |
| 10 | (R/S)-$CHCH_3$ | $CH_2CH_2$ | O | Cl | H | 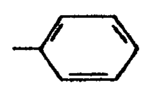 | H | $C_2H_5$ | |

<u>Tabelle 7</u> - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 11 | (S)-$CHCH_3$ | $CH_2CH_2$ | O | Cl | H | 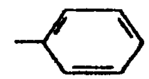 | $CH_3$ | $CH_3$ | (amorph) |
| 12 | $-(CH_2)_3-$ | $CH_2CH_2$ | O | Cl | H | 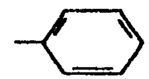 | H | $C_2H_5$ | 146 °C |
| 13 | $-(CH_2)_3-$ | $CH_2CH_2$ | O | Cl | H | 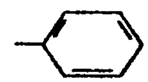 | H | $CH_3$ | 159 °C |
| 14 | $-(CH_2)_3-$ | $-(CH_2)_3-$ | O | Cl | H | 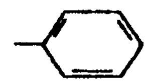 | H | $CH_3$ | 154 °C |
| 15 | (R)-$CHCH_3$ | $CH_2CH_2$ | O | $OCH_3$ | H | 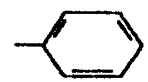 | H | $C_2H_5$ | (amorph) |
| 16 | (R/S)-CH-$CH_2$-<br>\|<br>$C_6H_5$ | $CH_2CH_2$ | O | Cl | H | 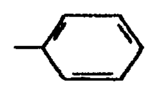 | H | $CH_3$ | 104 °C |
| 17 | (R/S)-CH-$CH_2$<br>\|<br>$C_6H_5$ | $CH_2CH_2$ | O | Cl | H | 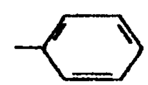 | H | $C_2H_5$ | 43 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 18 | (R/S)-CH-$CH_2$ with $C_6H_5$ on CH | $-(CH_2)_3-$ | O | Cl | H | 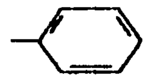 | H | $CH_3$ | 41 °C |
| 19 | (S)-$CHCH_3$ | $CH_2CH_2$ | S | Cl | H | 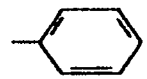 | H | $C_2H_5$ | 55 °C |
| 20 | $CH_2$ | $CH_2CH_2$ | O | Cl | H | 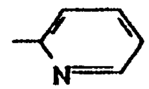 | H | $CH_3$ | 137 °C |
| 21 | $CH_2$ | $CH_2CH_2$ | O | Cl | H | 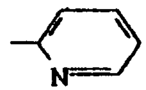 | H | $C_2H_5$ | 125 °C |
| 22 | $CH_2$ | $-(CH_2)_3-$ | O | Cl | H | 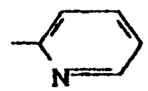 | H | $C_2H_5$ | 134 °C |
| 23 | $CH_2CH_2$ | $CH_2CH_2$ | O | Cl | H | 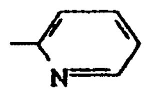 | H | $CH_3$ | 143 °C |
| 24 | $CH_2CH_2$ | $CH_2CH_2$ | O | Cl | H | 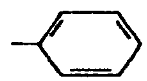 | H | $C_2H_5$ | 147 °C |

**Tabelle 7** - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 25 | $CH_2CH_2$ | $-(CH_2)_3-$ | O | Cl | H | 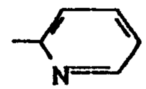 | H | $CH_3$ | 157 °C |
| 26 | $CH_2$ | $CH_2CH_2$ | O | Cl | H | 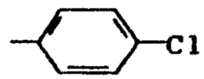 | H | $CH_3$ | 103 °C |
| 27 | (S)-$CHCH_3$ | $-(CH_2)_3-$ | O | Cl | H | 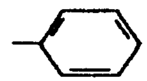 | H | $C_2H_5$ | 69 °C |
| 28 | (S)-$CHCH_3$ | $-(CH_2)_3-$ | O | Cl | H | 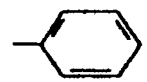 | H | $C_4H_9$ | 53 °C |
| 29 | $CH_2$ | $-(CH_2)_3-$ | O | Cl | H | 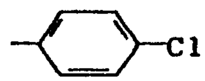 | H | $CH_3$ | 194 °C |
| 30 | $CH_2$ | $CH_2CH_2$ | O | Cl | H | 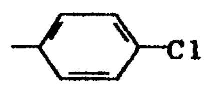 | H | $C_2H_5$ | 196 °C |
| 31 | $CH_2CH_2$ | $CH_2CH_2$ | O | Cl | H | 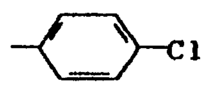 | H | $CH_3$ | 196 °C |
| 32 | $CH_2CH_2$ | $CH_2CH_2$ | O | Cl | H | 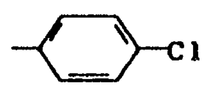 | H | $C_2H_5$ | 196 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 33 | $CH_2CH_2$ | $-(CH_2)_3-$ | O | Cl | H | 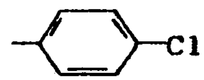 | H | $CH_3$ | 197 °C |
| 34 | (S)-$CHCH_3$ | $CH_2CH_2$ | O | $SCH(CH_3)_2$ | H | 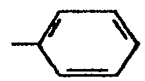 | H | $C_2H_5$ | $n_D^{20}$ = 1,5625 |
| 35 | (S)-$CHCH_3$ | $CH_2CH_2$ | O | $SC_2H_5$ | H | 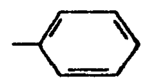 | H | $C_2H_5$ | (amorph) |
| 36 | (S)-$CHCH_3$ | $CH_2CH_2$ | O | $OC_2H_5$ | H | 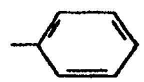 | H | $C_2H_5$ | (amorph) |
| 37 | (S)-$CHCH_3$ | $CH_2CH_2$ | O | $OCH(CH_3)_2$ | H | 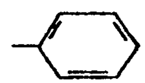 | H | $C_2H_5$ | (amorph) |
| 38 | (S)-$CHCH_3$ | $CH_2CH_2$ | O | Cl | $CH_3$ | 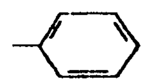 | H | $C_2H_5$ | (amorph) |
| 39 | (S)-$CHCH_3$ | $CH_2CH_2$ | O | $NHCH_3$ | H | 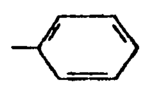 | H | $C_2H_5$ | (amorph) |
| 40 | $CH_2$ | $CH_2CH_2$ | O | Cl | H | 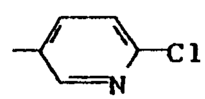 | H | $C_2H_5$ | 176 °C |

<u>Tabelle 7</u> - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 41 | $CH_2$ | $CH_2CH_2$ | O | Cl | H | 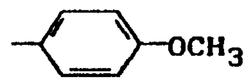 | H | $CH_3$ | 172 °C |
| 42 | $CH_2$ | $CH_2CH_2$ | O | Cl | H | 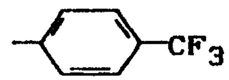 | H | $CH_3$ | 219 °C |
| 43 | $CH_2$ | $CH_2CH_2$ | O | Cl | H | 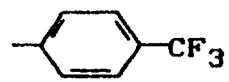 | H | $C_2H_5$ | 198 °C |
| 44 | $CH_2$ | $-(CH_2)_3-$ | O | Cl | H | 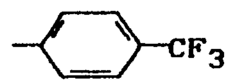 | H | $CH_3$ | 214 °C |
| 45 | $CH_2$ | $CH_2CH_2$ | O | Cl | H | 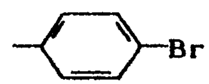 | H | $CH_3$ | 184 °C |
| 46 | $CH_2$ | $CH_2CH_2$ | O | Cl | H | 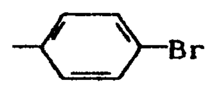 | H | $C_2H_5$ | 191 °C |
| 47 | $CH_2$ | $-(CH_2)_3-$ | O | Cl | H | 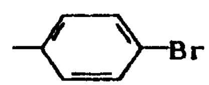 | H | $CH_3$ | 205 °C |
| 48 | $CH_2$ | $CH_2CH_2$ | O | Cl | H | 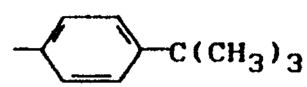 | H | $CH_3$ | 176 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 49 | $CH_2$ | $CH_2CH_2$ | O | Cl | H | 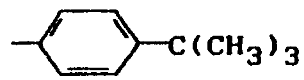 | H | $C_2H_5$ | 165 °C |
| 50 | $CH_2$ | $-(CH_2)_3-$ | O | Cl | H | 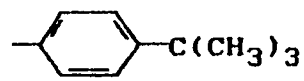 | H | $CH_3$ | 176 °C |
| 51 | $CH_2$ | $CH_2CH_2$ | O | Cl | H | 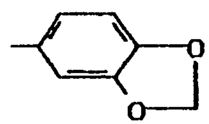 | H | $CH_3$ | 178 °C |
| 52 | $CH_2$ | $CH_2CH_2$ | O | Cl | H | 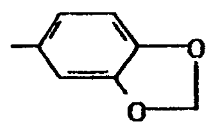 | H | $C_2H_5$ | 178 °C |
| 53 | $CH_2$ | $-(CH_2)_3-$ | O | Cl | H | 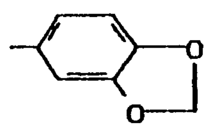 | H | $CH_3$ | 182 °C |
| 54 | $CH_2CH_2$ | $CH_2CH_2$ | O | Cl | H | 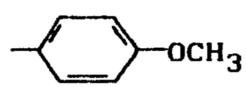 | H | $CH_3$ | 181 °C |
| 55 | $CH_2CH_2$ | $CH_2CH_2$ | O | Cl | H | 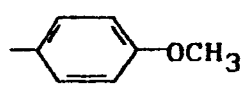 | H | $C_2H_5$ | 182 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 56 | $-CH_2CH-$<br>$CH_3$ | $CH_2CH_2$ | O | Cl | H | 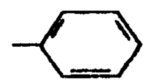 | H | $CH_3$ | 136 °C |
| 57 | $-CH_2CH-$<br>$CH_3$ | $CH_2CH_2$ | O | Cl | H | 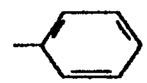 | H | $C_2H_5$ | 138 °C |
| 58 | $-CH_2CH-$<br>$CH_3$ | $-(CH_2)_3-$ | O | Cl | H | 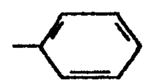 | H | $CH_3$ | 135 °C |
| 59 | $-CHCH_2CH_2$<br>$CH_3$ | $CH_2CH_2$ | O | Cl | H | 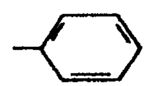 | H | $CH_3$ | (amorph) |
| 60 | $-CHCH_2CH_2$<br>$CH_3$ | $CH_2CH_2$ | O | Cl | H | 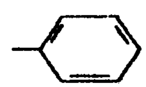 | H | $C_2H_5$ | (amorph) |
| 61 | $CH_2CH_2$ | $-(CH_2)_3-$ | O | Cl | H | 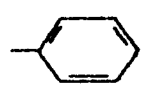 | H | $CH_3$ | (amorph) |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 62 | $CH_2$ | $CH_2CH_2$ | O | Cl | H | 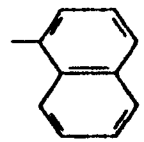 | H | $CH_3$ | 154 °C |
| 63 | $CH_2$ | $CH_2CH_2$ | O | Cl | H | 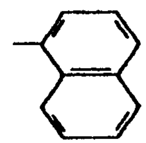 | H | $C_2H_5$ | 160 °C |
| 64 | $CH_2$ | $-(CH_2)_3-$ | O | Cl | H | 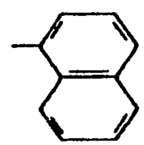 | H | $CH_3$ | 152 °C |
| 65 | $CH_2$ | $CH_2CH_2$ | O | $OCH_3$ | H | 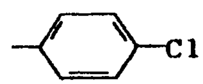 | H | $C_2H_5$ | 99 °C |
| 66 | (S)-$CHCH_3$ | $CH_2CH_2$ | O | $NH_2$ | H | 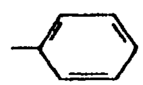 | H | $C_2H_5$ | (amorph) |
| 67 | $CH_2$ | $-(CH_2)_3-$ | O | Cl | H | 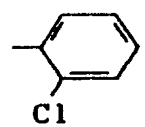 | H | $CH_3$ | 165 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 68 | $CH_2$ | $-(CH_2)_3-$ | O | Cl | H | 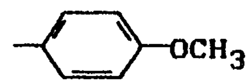 | H | $CH_3$ | 176 °C |
| 69 | $CH_2CH_2$ | $-(CH_2)_3-$ | O | Cl | H | 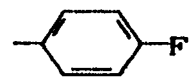 | H | $CH_3$ | 212 °C |
| 70 | $CH_2CH_2$ | $-(CH_2)_3-$ | O | Cl | H | 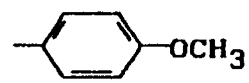 | H | $CH_3$ | 187 - 190 °C |
| 71 | $CH_2CH_2$ | $-(CH_2)_3-$ | O | Cl | H | 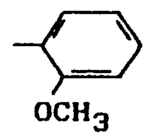 | H | $CH_3$ | 138 - 140 °C |
| 72 | $CH_2CH_2$ | $-(CH_2)_3-$ | O | Cl | H | 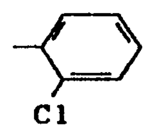 | H | $CH_3$ | 177 °C |
| 73 | (R/S)-$CHCH_3$ | $-(CH_2)_3-$ | O | Cl | H | 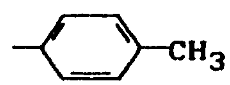 | H | $CH_3$ | 78 - 80 °C |
| 74 | (R/S)-$CHCH_3$ | $-(CH_2)_3-$ | O | Cl | H | 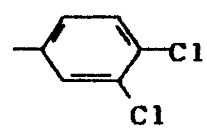 | H | $CH_3$ | 86 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 75 | (R/S)-$CHCH_3$ | $-(CH_2)_3-$ | O | Cl | H | 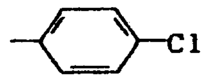 | H | $CH_3$ | 90 - 94 °C |
| 76 | $CH_2$ | $-(CH_2)_3-$ | O | $OCH_3$ | H | 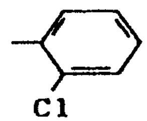 | H | $CH_3$ | 74 °C |
| 77 | $CH_2$ | $-(CH_2)_3-$ | O | $OCH_3$ | H | 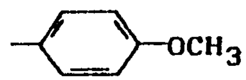 | H | $CH_3$ | 65 °C |
| 78 | $CH_2CH_2$ | $-(CH_2)_3-$ | O | $OCH_3$ | H | 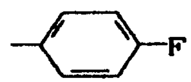 | H | $CH_3$ | 100 °C |
| 79 | (R/S)-$CHCH_3$ | $-(CH_2)_3-$ | O | Cl | H | 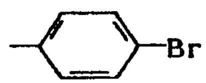 | H | $CH_3$ | 95 - 96 °C |
| 80 | (R/S)-$CHCH_3$ | $-(CH_2)_3-$ | O | Cl | H | 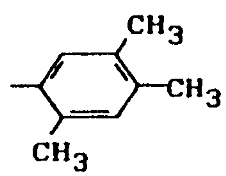 | H | $CH_3$ | 72 - 74 °C |
| 81 | (R/S)-$CHCH_3$ | $-(CH_2)_3-$ | O | Cl | H | 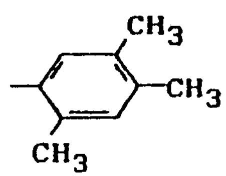 | H | $CH_3$ | 117 -120 °C |

<u>Tabelle 7</u> - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 82 | (R/S)-$CHCH_3$ | $-(CH_2)_3-$ | O | $OCH_3$ | H | 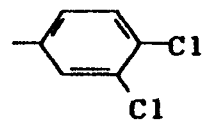 | H | $CH_3$ | $n_D^{21}$ = 1.5759 |
| 83 | (R/S)-$CHCH_3$ | $-(CH_2)_3-$ | O | $OCH_3$ | H | 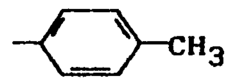 | H | $CH_3$ | 72 - 74 °C |
| 84 | $CH_2CH_2$ | $-(CH_2)_3-$ | O | Cl | H | 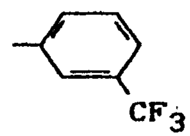 | H | $CH_3$ | 185 - 186 °C |
| 85 | (R/S)-$CHCH_3$ | $-(CH_2)_3-$ | O | $OCH_3$ | H | 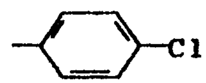 | H | $CH_3$ | $n_D^{21}$ = 1.5650 |
| 86 | $CH_2CH_2$ | $-(CH_2)_3-$ | O | Cl | H | 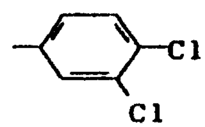 | H | $CH_3$ | 200 - 202 °C |
| 87 | (R/S)-$CHCH_3$ | $-(CH_2)_3-$ | O | Cl | H | 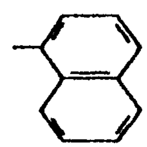 | H | $CH_3$ | 60 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 88 | (R/S)-$CHCH_3$ | $CH_2CH_2$ | O | Cl | H | 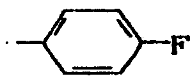 | H | $C_2H_5$ | $n_D^{21}$ = 1.5621 |
| 89 | $CH_2$ | $-(CH_2)_3-$ | O | Cl | H | 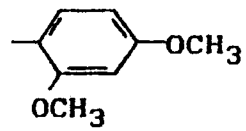 | H | $CH_3$ | 131 - 133 °C |
| 90 | $CH_2CH_2$ | $CH_2CH_2$ | O | Cl | H | 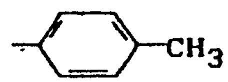 | H | $C_2H_5$ | 203 °C |
| 91 | $CH_2CH_2$ | $CH_2CH_2$ | O | Cl | H | 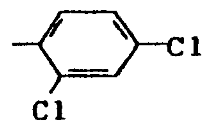 | H | $C_2H_5$ | 201 °C |
| 92 | (R/S)-$CHCH_3$ | $CH_2CH_2$ | O | Cl | H | 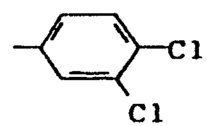 | H | $C_2H_5$ | 80 - 84 °C |
| 93 | (R/S)-$CHCH_3$ | $CH_2CH_2$ | O | Cl | H | 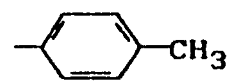 | H | $C_2H_5$ | $n_D^{21}$ = 1.5700 |
| 94 | $CH_2$ | $CH_2CH_2$ | O | Cl | H | 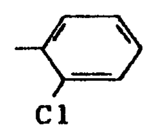 | H | $C_2H_5$ | 160 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 95 | (R/S)-$CHCH_3$ | $CH_2CH_2$ | O | Cl | H | 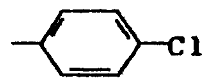 | H | $C_2H_5$ | 58 °C |
| 96 | $CH_2CH_2$ | $CH_2CH_2$ | O | Cl | H | 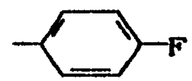 | H | $C_2H_5$ | 208 - 211 °C |
| 97 | $CH_2$ | $CH_2CH_2$ | O | Cl | H | 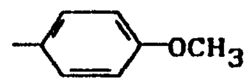 | H | $C_2H_5$ | 195 °C |
| 98 | $CH_2CH_2$ | $CH_2CH_2$ | O | Cl | H | 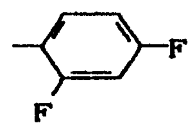 | H | $C_2H_5$ | 114 °C |
| 99 | $CH_2CH_2$ | $CH_2CH_2$ | O | Cl | H | 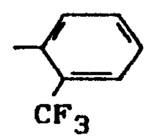 | H | $C_2H_5$ | 194 °C |
| 100 | $CH_2CH_2$ | $CH_2CH_2$ | O | Cl | H | 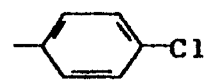 | H | $C_2H_5$ | 199 °C |
| 101 | (R/S)-$CHCH_3$ | $CH_2CH_2$ | O | Cl | H | 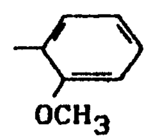 | H | $C_2H_5$ | 107 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 102 | (R/S)-$CHCH_3$ | $CH_2CH_2$ | O | Cl | H | 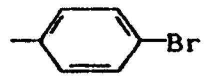 | H | $C_2H_5$ | 47 °C |
| 103 | (R/S)-$CHCH_3$ | $CH_2CH_2$ | O | Cl | H | 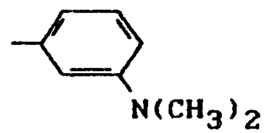 | H | $C_2H_5$ | $n_D^{21}$ = 1.5721 |
| 104 | (R/S)-$CHCH_3$ | $CH_2CH_2$ | O | Cl | H | 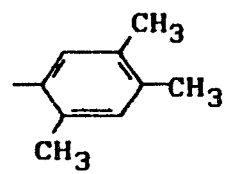 | H | $C_2H_5$ | 125 - 127 °C |
| 105 | $CH_2$ | $CH_2CH_2$ | O | Cl | H | 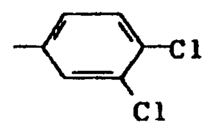 | H | $C_2H_5$ | 182 °C |
| 106 | $CH_2CH_2$ | $CH_2CH_2$ | O | Cl | H | 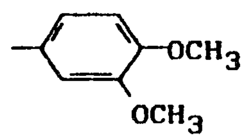 | H | $OCH_3$ | 170 °C |
| 107 | $CH_2CH_2$ | $CH_2CH_2$ | O | Cl | H | 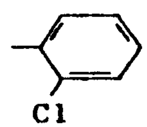 | H | $C_2H_5$ | 190 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 108 | $CH_2CH_2$ | $CH_2CH_2$ | O | Cl | H | 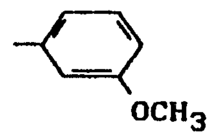 | H | $C_2H_5$ | 175 °C |
| 109 | $CH_2CH_2$ | $CH_2CH_2$ | O | $OCH_3$ | H | 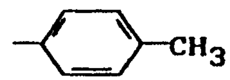 | H | $C_2H_5$ | 245 °C |
| 110 | (R/S)-$CHCH_3$ | $-(CH_2)_3-$ | O | $OCH_3$ | H | 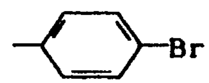 | H | $CH_3$ | $n_D^{21}$ = 1.5830 |
| 111 | (R/S)-$CHCH_3$ | $CH_2CH_2$ | O | $OCH_3$ | H | 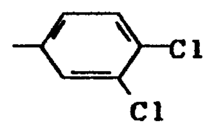 | H | $C_2H_5$ | $n_D^{21}$ = 1.5762 |
| 112 | (R/S)-$CHCH_3$ | $CH_2CH_2$ | O | $OCH_3$ | H | 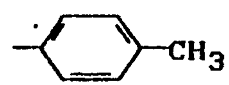 | H | $C_2H_5$ | $n_D^{21}$ = 1.5560 |
| 113 | (R/S)-$CHCH_3$ | $-(CH_2)_3-$ | O | $OCH_3$ | H | 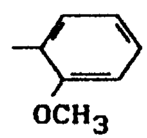 | H | $CH_3$ | $n_D^{21}$ = 1.5638 |
| 114 | $CH_2CH_2$ | $-(CH_2)_3-$ | O | $OCH_3$ | H | 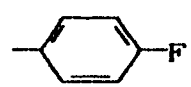 | H | $CH_3$ | 95 - 96 °C |

Tabelle 7 - Fortsetzung

| Beisp.-Nr. | $A^1$ | $A^2$ | Q | $R^1$ | $R^2$ | $R^3$ | $R^4$ | $R^5$ | Schmelzpunkt bzw. Brechungsindex |
|---|---|---|---|---|---|---|---|---|---|
| 115 | (R/S)-$CHCH_3$ | $-(CH_2)_3-$ | O | $OCH_3$ | H | 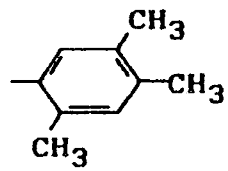 | H | $CH_3$ | 76 °C |
| 116 | (R/S)-$CHCH_3$ | $CH_2CH_2$ | O | $OCH_3$ | H | 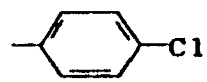 | H | $C_2H_5$ | 250 °C |
| 117 | (R/S)-$CHCH_3$ | $-(CH_2)_3-$ | O | Cl | H | 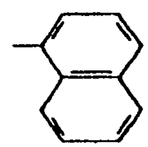 | H | $CH_3$ | 88 °C |
| 118 | $C(CH_3)_2$ | $-(CH_2)_3-$ | O | Cl | H | 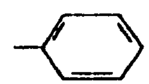 | H | $CH_3$ | 138 °C |

Beispiel 119

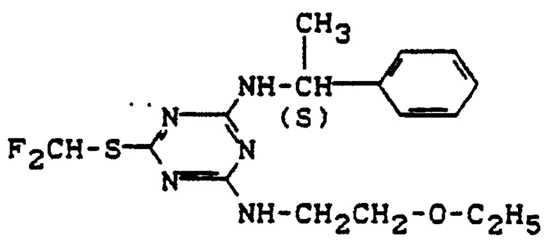

(Verfahren (d))

Eine Mischung aus 8,0 g (0,025 Mol) (S)-2-Mercapto-4-(2-ethoxy-ethylamino)-6-(1-phenyl-ethylamino)-s-triazin, 4,0 g (0,10 Mol) Natriumhydroxid, 6 ml Wasser und 17 ml Isopropanol wird 30 Minuten auf 56 °C erhitzt und dann auf 20 °C abgekühlt. In diese Mischung wird dann 3 Stunden lang Chlordifluormethan eingeleitet. Anschließend wird mit 200 ml Wasser verdünnt, mit Methylenchlorid geschüttelt, die organische Phase abgetrennt, mit Wasser gewaschen, mit Natriumsulfat getrocknet und filtriert. Vom Filtrat wird das Lösungsmittel im Wasserstrahlvakuum sorgfältig abdestilliert.

Man erhält 6,1 g (66 % der Theorie) (S)-2-Difluormethylthio-4-(2-ethoxy-ethylamino)-6-(1-phenyl-ethylamino)-s-triazin als öligen Rückstand vom Brechungsindex $n_D^{25}$ = 1,5518.

Beispiel 120

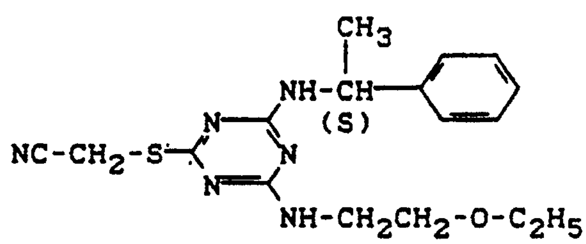

(Verfahren (d))

Eine Mischung aus 8,0 g (0,025 Mol) (S)-2-Mercapto-4-(2-ethoxy-ethylamino)-6-(1-phenyl-ethylamino)-s-triazin, 4,0 g (0,10 Mol) Natriumhydroxid, 6 ml Wasser und 17 ml Isopropanol wird 30 Minuten auf 56 °C erhitzt und dann auf 20 °C abgekühlt. Nach Zugabe von 3,0 g (0,025 Mol) Bromacetoniril wird das Reaktionsgemisch 4 Stunden bei 20 °C gerührt und anschließend wie bei Beispiel 119 beschrieben aufgearbeitet.

Man erhält 5,2 g (56 % der Theorie) (S)-2-Cyanomethylthio-4-(2-ethoxy-ethylamino)-6-(1-phenyl-ethylamino)-s-triazin als öligen Rückstand.

Ausgangsstoffe der Formel (II)

Beispiel (II-1)

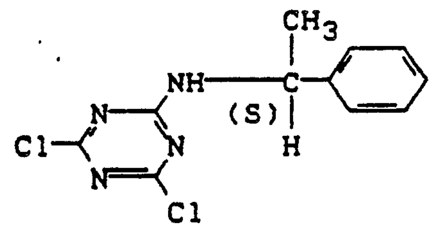

Eine Lösung von 24,2 g (0,2 Mol) (S)-1-Phenyl-ethylamin in 50 ml Tetrahydrofuran wird unter Rühren zu einer auf -30 °C bis -40 °C gekühlten Lösung von 18,4 g (0,1 Mol) 2,4,6-Trichlor-s-triazin (Cyanurchlorid) in 100 ml Tetrahydrofuran tropfenweise gegeben. Das Reaktionsgemisch wird weitere 45 Minuten bei -30 °C bis -40 °C gerührt und dann kalt abgesaugt. Vom Filtrat wird das Lösungsmittel im Wasserstrahlvakuum sorgfältig abdestilliert. Der zurückbleibende ölige Rückstand kristallisiert nach längerem Stehen.

Man erhält 25 g (93 % der Theorie) (S)-2,4-Dichlor-6-(1-phenyl-ethylamino)-s-triazin vom Schmelzpunkt 75 °C.

Analog Beispiel (II-1) können die in Tabelle 1 aufgeführten Verbindungen der Formel (II) hergestellt werden.

Ausgangsstoffe der Formel (IV)

Beispiel (IV-1)

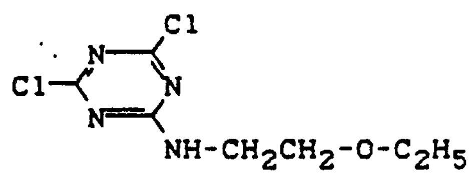

Eine Lösung von 9,7 g (0,11 Mol) 2-Ethoxy-ethylamin und 11,0 g (0,11 Mol) Triethylamin in 100 ml Tetrahydrofuran wird unter Rühren zu einer auf -30 °C bis -40 °C gekühlten Lösung von 20 g (0,11 Mol) 2,4,6-Trichlor-s-triazin (Cyanurchlorid) in 100 ml Tetrahydrofuran tropfenweise gegeben. Das Reakionsgemisch wird 60 Minuten bei -30 °C bis -40 °C gerührt und abgesaugt. Vom Filtrat wird das Lösungsmittel im Wasserstrahlvakuum sorgfältig abdestilliert. Der zurückbleibende Rückstand kristallisiert nach längerem Stehen.

Man erhält 21,2 g (84 % der Theorie) 2,4-Dichlor-6-(2-ethoxy-ethylamino)-s-triazin vom Schmelzpunkt 77 °C.

Analog Beispiel (IV-1) können die in Tabelle 3 aufgeführten Verbindungen der Formel (IV) hergestellt werden.

Ausgangsstoffe der Formel (VII)

Beispiel (VII-1)

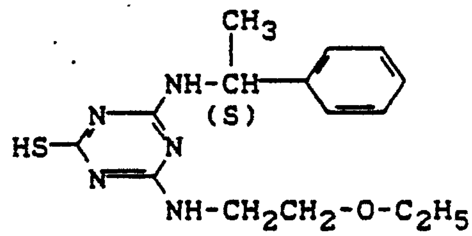

Eine Mischung aus 32,1 g (0,10 Mol) (S)-2-Chlor-4-(2-ethoxy-ethylamino)-6-(1-phenyl-ethylamino)-s-triazin, 8,0 g (0,105 Mol) Thioharnstoff und 50 ml Dioxan wird unter Rückfluß zum Sieden erhitzt, nach 1 bis 2 Minuten auf ca. 80 °C abgekühlt und nach Zugabe von 150 g 2N-Natronlauge noch 1 Stunde auf 100 °C erhitzt. Nach dem Abkühlen wird filtriert, das Filtrat mit Ammoniumchlorid neutralisiert, das kristallin angefallende Produkt durch Absaugen isoliert, mit Wasser gewaschen, in Chloroform gelöst, mit Magnesiumsulfat getrocknet und filtriert. Vom Filtrat wird das Lösungsmittel im Wasserstrahlvakuum sorgfältig abdestilliert.

Man erhält 29,4 g (92 % der Theorie) (S)-2-Mercapto-4-(2-ethoxy-ethylamino)-6-(1-phenyl-ethylamino)-s-triazin als kristallinen Rückstand vom Schmelzpunkt 53 °C.

Analog Beispiel (VII-1) können die in Tabelle 6 aufgeführten Verbindungen der Formel (VII) hergestellt werden.

Anwendungsbeispiel

Im folgenden Anwendungsbeispiel wird die Verbindung nachstehender Formel als Vergleichssubstanz verwendet :

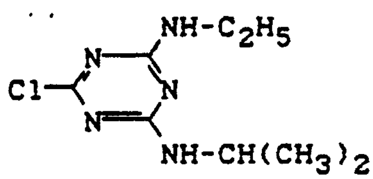

(A)

2-Chlor-4-ethylamin-6-isopropylamino-s-triazin (Atrazin).

Beispiel A

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß den Herstellungsbeispielen (1) bzw. (2) und (3) bessere Selektivität und erheblich stärkere Wirkung als die Vergleichssubstanz (A).

## Ansprüche

1. Substituierte Triazine der allgemeinen Formel (I)

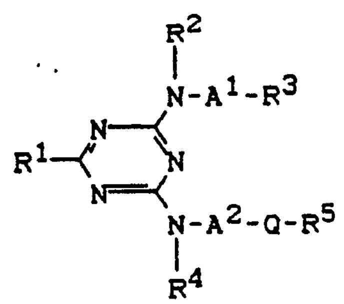

(I)

in welcher
$A^1$ für gegebenenfalls verzweigtes und gegebenenfalls durch Aryl substituiertes Alkandiyl ("Alkylen") steht,
$A^2$ für gegebenenfalls verzweigtes Alkandiyl ("Alkylen") steht,
Q für Sauerstoff, Schwefel, NH oder N-Alkyl steht,
$R^1$ für Wasserstoff, Hydroxy, Nitro, Cyano, Cyanamino, Azido, Halogen, Alkoxy, Alkylthio (welches gegebenenfalls durch Halogen oder Cyano substituiert ist), Alkyl, Alkoxycarbonyl, Aminocarbonyl, Alkylaminocarbonyl, Dialkylaminocarbonyl, Amino, Alkylamino oder Dialkylamino steht,
$R^2$ für Wasserstoff, Alkyl, Alkenyl oder Alkinyl steht,
$R^3$ für gegebenenfalls substituiertes Aryl oder Heteroaryl steht,
$R^4$ für Wasserstoff, Alkyl, Alkenyl oder Alkinyl steht und
$R^5$ für Wasserstoff oder Alkyl steht.

2. Verbindungen der Formel (I) gemäß Anspruch 1, dadurch gekennzeichnet, daß darin
$A^1$ für gegebenenfalls verzweigtes und gegebenenfalls durch Phenyl substituiertes Alkandiyl ("Alkylen") mit 1 bis 4 Kohlenstoffatomen steht,
$A^2$ für gegebenenfalls verzweigtes Alkandiyl ("Alkylen") mit 1 bis 4 Kohlenstoffatomen steht,
Q für Sauerstoff, Schwefel, NH oder N-($C_1$-$C_4$-Alkyl) steht,
$R^1$ für Wasserstoff, Hydroxy, Nitro, Cyano, Cyanamino, Azido, Fluor, Chlor, Brom, Iod, $C_1$-$C_4$-Alkoxy, $C_1$-$C_4$-Alkylthio (welches gegebenenfalls durch Cyano, Fluor und/oder Chlor substituiert ist), $C_1$-$C_4$-Alkyl, $C_1$-$C_4$-Alkoxy-car bonyl, Aminocarbonyl, $C_1$-$C_4$-Alkyl-amino-carbonyl, Di-($C_1$-$C_4$-alkyl)-amino-carbonyl, Amino, $C_1$-$C_4$-Alkylamino oder Di-($C_1$-$C_4$-alkyl)-amino steht,
$R^2$ für Wasserstoff, $C_1$-$C_4$-Alkyl, $C_2$-$C_4$-Alkenyl oder $C_2$-$C_4$-Alkinyl steht,
$R^3$ für eine gegebenenfalls durch Fluor, Chlor, Brom, Iod, Cyano, Nitro, $C_1$-$C_4$-Alkyl (welches gegebenenfalls durch Fluor und/oder Chlor substituiert ist), $C_1$-$C_4$-Alkoxy oder $C_1$-$C_2$-Alkylendioxy (welche gegebenenfalls durch Fluor und/oder Chlor substituiert sind), $C_1$-$C_4$-Alkylthio, $C_1$-$C_4$-Alkyl-sulfinyl oder $C_1$-$C_4$-Alkylsulfonyl (welche gegebenenfalls durch Fluor und/oder Chlor substituiert sind), Di-($C_1$-$C_2$-alkyl)-amino und/oder

durch $C_1$-$C_4$-Alkoxy-carbonyl substituierte aromatische bzw. heteroaromatische Gruppierung aus der Reihe Phenyl, Naphthyl, Pyridyl, Chinolinyl, Isochinolinyl, Pyrimidinyl, Furyl, Thienyl, Pyrrolyl, Pyrazolyl oder Imidazolyl steht,

$R^4$ für Wasserstoff, $C_1$-$C_4$-Alkyl, $C_2$-$C_4$-Alkenyl oder $C_2$-$C_4$-Alkinyl steht und

$R^5$ für Wasserstoff oder $C_1$-$C_4$-Alkyl steht.

3. Verbindungen der Formel (I) gemäß Anspruch 1, dadurch gekennzeichnet, daß darin

$A^1$ für gegebenenfalls verzweigtes und gegebenenfalls durch Phenyl substituiertes Alkandiyl ("Alkylen") mit 1 bis 3 Kohlenstoffatomen steht,

$A^2$ für gegebenenfalls verzweigtes Alkandiyl ("Alkylen") mit 1 bis 3 Kohlenstoffatomen steht,

Q für Sauerstoff, Schwefel, NH oder N-$CH_3$ steht,

$R^1$ für Cyanamino, Azido, Fluor, Chlor, Brom, Methoxy, Ethoxy, Methylthio, Ethylthio, Difluormethylthio, Cyanomethylthio, Methyl, Amino, Methylamino oder Dimethylamino steht,

$R^2$ für Wasserstoff oder Methyl steht,

$R^3$ für gegebenenfalls durch Fluor, Chlor, Brom, Cyano, Nitro, Methyl, Ethyl, Methoxy und/oder Ethoxy substituiertes Phenyl oder Naphthyl, fü gegebenenfalls durch Fluor, Chlor, Brom, Cyano, Nitro, Methyl, Ethyl, Methoxy und/oder Ethoxy substituiertes Pyridyl oder für gegebenenfalls durch Fluor, Chlor, Brom, Methyl und/oder Ethyl substituiertes Furyl steht,

$R^4$ für Wasserstoff oder Methyl steht und

$R^5$ für $C_1$-$C_3$-Alkyl steht.

4. Verfahren zur Herstellung von substituierten Triazinen der allgemeinen Formel (I) gemäß Anspruch 1, dadurch gekennzeichnet, daß man

(a) halogenierte Triazine der allgemeinen Formel (II)

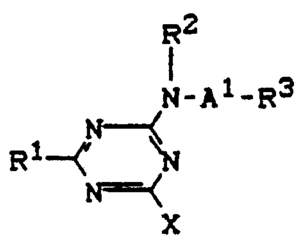

(II)

in welcher

$A^1$, $R^1$, $R^2$ und $R^3$ die in Anspruch 1 angegebenen Bedeutungen haben und

X für Halogen steht,

mit Aminoverbindungen der allgemeinen Formel (III)

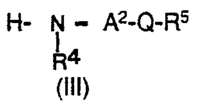

(III)

in welcher

$A^2$, Q, $R^4$ und $R^5$ die in Anspruch 1 angegebenen Bedeutungen haben,

gegebenenfalls in Gegenwart eines Säureakzeptors und gegebenenfalls in Gegenwart eines Verdünnungsmittels umsetzt, oder

(b) halogenierte Triazine der allgemeinen Formel (IV)

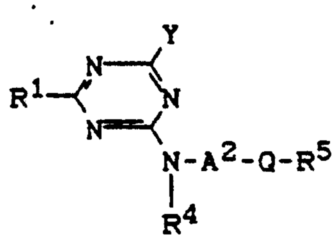

(IV)

in welcher

$A^2$, Q, $R^1$, $R^4$ und $R^5$ die in Anspruch 1 angegebenen Bedeutungen haben und

Y für Halogen steht,

mit Aminoverbindungen der allgemeinen Formel (V)

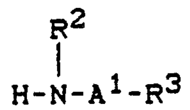

(V)

in welcher
$A^1$, $R^2$ und $R^3$ die in Anspruch 1 angegebenen Bedeutungen haben,
gegebenenfalls in Gegenwart eines Säureakzeptors und gegebenenfalls in Gegenwart eines Verdünnungsmittels umsetzt, oder
- für den Fall, daß $R^1$ für Amino, Alkylamino, Dialkylamino, Alkoxy oder Alkylthio steht -
(c) halogenierte Triazine der allgemeinen Formel (VI)

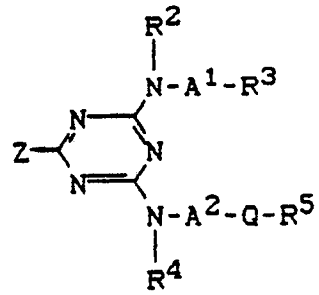

(VI)

in welcher
$A^1$, $A^2$, Q, $R^2$, $R^3$, $R^4$ und $R^5$ die in Anspruch 1 angegebenen Bedeutungen haben und
Z für Halogen steht,
mit Ammoniak, Alkylaminen, Dialkylaminen, Alkanolen bzw. Alkanthiolen oder mit deren Alkalimetallsalzen gegebenenfalls in Gegenwart eines Säureakzeptors und gegebenenfalls in Gegenwart eines Verdünnungsmittels umsetzt, oder
- für den Fall, daß $R^1$ für Alkylthio (welches gegebenenfalls durch Halogen oder Cyano substituiert ist) steht -
(d) Mercaptotriazine der allgemeinen Formel (VII)

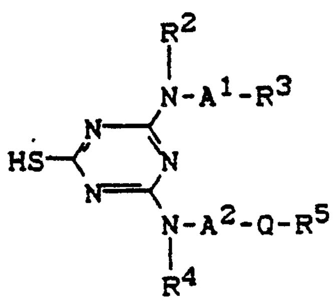

(VII)

in welcher
$A^1$, $A^2$, Q, $R^2$, $R^3$, $R^4$ und $R^5$ die in Anspruch 1 angegebenen Bedeutungen haben,
mit Halogenverbindungen der allgemeinen Formel (VIII)
$Z^1 - R^6$ (VIII)
in welcher
$R^6$ für Alkyl (welches gegebenenfalls durch Halogen oder Cyano substituiert ist) steht und
$Z^1$ für Halogen steht,
gegebenenfalls in Gegenwart eines Säureakzeptors und gegebenenfalls in Gegenwart eines Verdünnungsmittels umsetzt.

5. Herbizide Mittel, gekennzeichnet durch einen Gehalt an mindesten einem substituierten Triazin der allgemeinen Formel (I) gemäß Anspruch 1.

6. Verwendung von substituierten Triazinen der allgemeinen Formel (I) gemäß Anspruch 1 zur Bekämpfung von unerwünschtem Pflanzenwachstum.

7. Verfahren zur Herstellung von herbiziden Mitteln, dadurch gekennzeichnet, daß man substituierte Triazine der allgemeinen Formel (I) gemäß Anspruch 1 mit Streckmitteln, und/oder oberflächenaktiven Mitteln vermischt.